(19) Europäisches Patentamt
European Patent Office
Office européen des brevets

(11) Veröffentlichungsnummer: **0 091 650**
**B1**

(12) # EUROPÄISCHE PATENTSCHRIFT

(45) Veröffentlichungstag der Patentschrift: **30.07.86**

(51) Int. Cl.⁴: B 01 D 53/34, F 23 J 15/00, B 01 D 53/14

(21) Anmeldenummer: **83103344.4**

(22) Anmeldetag: **06.04.83**

(54) **Verfahren zur Absorption von Schwefeldioxid.**

(30) Priorität: **10.04.82 DE 3213389**

(43) Veröffentlichungstag der Anmeldung: **19.10.83 Patentblatt 83/42**

(45) Bekanntmachung des Hinweises auf die Patenterteilung: **30.07.86 Patentblatt 86/31**

(84) Benannte Vertragsstaaten: **AT BE CH DE FR GB IT LI LU NL SE**

(56) Entgegenhaltungen:
**DE-A- 2 615 339**
**GMELINS "Handbuch der anorganischen Chemie", 8. Auflage, "Chlor" Ergänzungsband, Teil B, Lieferung 2 1969, VERLAG CHEMIE, Weinheim/Bergstrasse, Seiten 400-405**
**Die Akte enthält technische Angaben, die nach dem Eingang der Anmeldung eingereicht wurden und die nicht in dieser Patentschrift enthalten sind.**

(73) Patentinhaber: **Kühne, Friedrich-Wilhelm Dr.**
**Paulinenstrasse 56**
**D-7107 Neckarsulm (DE)**

(72) Erfinder: **Kühne, Friedrich-Wilhelm Dr.**
**Paulinenstrasse 56**
**D-7107 Neckarsulm (DE)**

(74) Vertreter: **Beil, Walter, Dr. et al**
**BEIL, WOLFF & BEIL Rechtsanwälte Adelonstrasse 58**
**D-6230 Frankfurt am Main 80 (DE)**

**EP 0 091 650 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft das in den Patentansprüchen beschriebene Verfahren zur Absorption von Schwefeldioxid mittels einer wäßrigen, alkalischen Lösung eines Absorptionsmittels auf Chlorit-Basis.

Die Absorption von Schwefeldioxid spielt eine bedeutende Rolle für die Rauchgasentschwefelung. Da beispielsweise nach der neuen deutschen Großfeuerungsanlagenverordnung (GFAVO) bei Kraftwerksbauten mit Kohlefeuerung der $SO_2$-Emissionswert 400 mg/m$^3$ Rauchgas nicht übersteigen darf, muß der überwiegende Teil des Rauchgases dieser Anlagen entschwefelt werden.

Die bekannten Verfahren zur Naßentschwefelung von Rauchgas unterscheiden sich hauptsächlich durch verschiedenartige Wäschertypen, Absorptionsmittel und Endprodukte.

In der Zeitschrift VGB Kraftwerkstechnik 61, Heft 10 (Oktober 1981), Seiten 849 bis 856 wurde von H. Gutberlet und S. Stappert über praktische Erfahrungen mit einer Rauchgasentschwefelungsanlage berichtet, in der das aus den Rauchgasen ausgewaschene Schwefeldioxid unter Verwendung von Weißkalkhydrat in Gips umgewandelt wird. Der auf diese Weise anfallende Gips soll von der Industrie weiterverarbeitet werden. Es bestehen jedoch bereits Abnahmeschwierigkeiten für diesen Gips.

Aus der DE-A-2 615 339 ist ein Verfahren zur Reinigung von Abluft oder Abwässern, die beispielsweise Schwefeldioxid enthalten, durch Behandlung mit Alkalichloriten oder Erdalkalichloriten in wäßriger Lösung bei pH-Werten über 9 bekannt. Bei diesem bekannten Verfahren wird z. B. Schwefeldioxid mittels einer wäßriger Lösung von Natriumchlorit, die durch Zusatz von Kalilauge auf einen pH-Wert von 12,4 eingestellt wurde, absorbiert. Die als Absorptionsmittel eingesetzte Natriumchloritlösung muß jeweils an Ort und Stelle frisch hergestellt werden, da ihre Lagerstabilität begrenzt ist. Sie besitzt mit einer $LD_{50}$ von 6,9 mg/kg Körpergewicht eine erhebliche Toxizität. Das bei dieser Absorption anfallende Produkt ist nicht besonders brauchbar.

Aus Gmelins Handbuch der anorganischen Chemie, Chlor-Ergänzungsband, Teil B, Lieferung 2, 1969, Seite 404 ist bekannt, daß sich bei der Einwirkung von $ClO_2$ auf $ClO_2^-$ ein Charge-Transfer-Komplex $ClO_2 \cdot ClO_2^-$ bildet, der die Lösung dunkelrot färbt. Dieser bekannte Charge-Transfer-Komplex wurde jedoch nicht weiter umgesetzt.

Der Erfindung liegt die Aufgabe zugrunde, ein neues Verfahren zur Absorption von Schwefeldioxid bereitzustellen, bei dem die mit dem Stand der Technik verbundenen Nachteile vermieden werden.

Diese Aufgabe wird erfindungsgemäß dadurch gelöst, daß man als Absorptionsmittel eine wäßrige Lösung eines Komplexes aus aktiviertem Sauerstoff und Chlorit mit der allgemeinen Formel $ClO_2^- \cdot nO_2$, worin n = 0,1 bis 0,25 bedeutet, verwendet.

Bei dem im erfindungsgemäßen Verfahren als Absorptionsmittel wirksamen Komplex aus aktiviertem Sauerstoff und Chlorit handelt es sich um einen neuen Komplex, dessen Existenz im Raman-Spektrum durch eine Bande bei 1 562 cm$^{-1}$ (für den aktivierten Sauerstoff) nachgewiesen wurde. Dieser Komplex entsteht nur über die nachstehend angegebene Reaktionsfolge und kann bei dem aus der DE-A-2 615 339 bekannten Verfahren, etwa durch Umsetzung der dort verwendeten alkalischen Chloritlösung mit Luftsauerstoff, nicht entstehen. Die Raman-Bande kann auch nicht gelöstem Sauerstoff, dessen Bindungslänge nicht bekannt ist, zugeschrieben werden, da eine alkalische Chloritlösung keine nennenswerten Mengen an gelöstem Sauerstoff enthält.

Das im erfindungsgemäßen Verfahren verwendete Absorptionsmittel weist gegenüber der bekannten Verwendung von Weißkalkhydrat eine erhöhte Absorptionskapazität auf, so daß der Entschwefelungsgrad des Rauchgases auf diese Weise wesentlich erhöht wird. Dabei bewirkt das erfindungsgemäß verwendete Absorptionsmittel einerseits aufgrund seiner oxidativen Eigenschaften eine Oxidation des absorbierten $SO_2$ zu Sulfat, welches irreversibel aus dem Absorptionsgleichgewicht entfernt wird, und andererseits aufgrund seines hohen pH-Wertes eine Beschleunigung von Reaktionen des $SO_2$ mit basischen Verbindungen zu Folgeprodukten. Als Produkt des erfindungsgemäßen Verfahrens fällt Natriumsulfat, insbesondere Glaubersalz, an das sich in Form von Glaubersalzbatterien im Sinne eine Recycling-Verfahrens zur Energiespeicherung verwenden läßt.

Die erfindungsgemäß als Absorptionsmittel eingesetzte wäßrige Lösung hat einen alkalischen pH-Wert, vorzugsweise einen pH-Wert von 11,0 bis 14,5, insbesondere von 11,0 bis 13,5.

Im erfindungsgemäßen Verfahren finden beim Einleiten von $SO_2$ in die vorstehende Lösung bei Raumtemperatur oder bei erhöhten Temperaturen die folgenden Reaktionen statt:

$$SO_2 + 4\ OH^- \longrightarrow SO^{2-} + 2e + 2H_2O\ (2x)$$

$$ClO_2^- + 4e + 2H_2O \longrightarrow Cl^- + 4OH^-$$

$$\overline{\hspace{10em}}$$

$$2\ SO_2 + 4\ OH^- + ClO_2^- \longrightarrow 2\ SO_4^{2-} + Cl^- + 2\ H_2O$$

Diese Reaktionen, die auch in größeren Verdünnungen des Absorptionsmittels ablaufen, lassen sich ramanspektroskopisch verfolgen. So findet also im erfindungsgemäßen Verfahren nicht nur eine Absorption von $SO_2$ aufgrund des hohen pH-Wertes der Absorptionslösung, sondern auch eine Oxidation von $SO_2$ zu Sulfat statt. Die Absorptionskapazität von 1 Liter der erfindungsgemäß verwendeten

Absorptionslösung mit dem pH-Wert von 11,0 bis 14,5 beträgt etwa 200 bis 220 g $SO_2$ durch Oxidation und etwa 400 g $SO_2$ durch Absorption.

Das erfindungsgemäß verwendete Absorptionsmittel aus einer wäßrigen Lösung eines Komplexes aus aktiviertem Sauerstoff und Chlorit mit der allgemeinen Formel $ClO_2^- \cdot nO_2$, worin n = 0,1 bis 0,25 bedeutet, kann hergestellt werden, indem man ein Chlorit, wie Natriumchlorit, in wäßriger Lösung mit einem Hypochlorit, wie Natriumhypochlorit umsetzt. Die Umsetzung erfolgt in einem Molverhältnis von Chlorit zu Hypochlorit wie 1 : 0,2 bis 1 : 0,3, vorzugsweise 1 : 0,25. In diesem Medium erfolgt innerhalb einer Stunde keine Veränderung der Edukte. Diese alkalische Lösung von Natriumchlorit und Natriumhypochlorit wird mit « Chlorylschwefelsäure » auf einen pH-Wert von 6,4 bis 6,8 titriert. Dabei bilden sich Chlordioxid und der bekannte Charge-Transfer-Komplex $(Cl_2O_4)^-$. Aus diesem Komplex wird sodann unter Zusatz von Peroxidcarbonaten oder Peroxidboraten, z. B. Natriumperborat oder Natriumpercarbonat, und anschließend Natriumperoxid Chlordioxid ausgetrieben. Dabei entsteht eine Matrix aus Chloritionen, die aktivierten Sauerstoff eingeschlossen enthält und dem Komplex mit der vorstehenden allgemeinen Formel entspricht.

Die vorstehende Reaktionsfolge kann wie folgt erläutert werden :

$$2\,ClO_2^- + OCl^- + 2H^+ \longrightarrow 2\,ClO_2 + Cl^- + H_2O \qquad (1)$$

$$ClO_2 + ClO_2^- \longrightarrow (Cl_2O_4)^- \qquad (2)$$

Die Hälfte der eingesetzten Chloritmenge wird gemäß Gleichung (1) in einem Redoxprozeß zu $ClO_2$ oxidiert. Das entstehende Chlordioxid bildet mit der zweiten Hälfte nichtoxidiertem Chlorit einen intensiv braunen Charge-Transfer-Komplex entsprechend Gleichung (2).

Das Maximum an Ausbeute wird nach 60 bis 90 Minuten erreicht. Anschließend versetzt man die Lösung — je nach künftiger Verwendung — mit geringen Mengen $Na_2CO_3 \times H_2O_2$ oder $NaBO_2 \times H_2O_2 \times 3H_2O$, wobei sich die Farbe nach gelb aufhellt. Durch das Peroxid wird ein Teil des gelösten Chlordioxids unter stürmischer Entwicklung von Sauerstoff wieder zu Chlorit reduziert. Nach weiteren 15 Minuten gibt man eine geringe Menge $Na_2O_2$ zur Lösung, die sich nun vollständig entfärbt, da restliches $ClO_2$ zu $ClO_2^-$ reduziert wird. Nunmehr erfolgt in sehr langsamer Reaktion, die mindestens 4 Wochen erfordert und üblicherweise mehr als 6 Wochen in Anspruch nimmt, die Bildung einer aktivierten Sauerstoff enthaltenden matrix aus Chloritionen unter gleichzeitiger Bildung von Hydroxylionen. Diese Reaktion kann nicht beschleunigt werden. Der pH-Wert erhöht sich bis auf 13,8. Verschiedene Versuche haben gezeigt, daß sich das Volumen über der Lösung auf den Sauerstoffgehalt auswirkt. Am günstigsten erwies sich ein Verhältnis von 2/3 Lösung zu 1/3 Gasvolumen.

Stöchiometrisch sollte man, von der gemäß Gleichung (1) gebildeten Menge Chlordioxid aus gesehen, ein Verhältnis von Chlorit zu Sauerstoff wie 4 : 1 erwarten. Hinzu kommt möglicherweise Sauerstoff, welcher sich durch disproporitonierung von Wasserstoffperoxid bildet. Das Verhältnis sollte sich also noch mehr zugunsten von Sauerstoff verschieben. Quantitative Sauerstoffbestimmungen haben aber gezeigt, daß so hohe Sauerstoff-Konzentrationen nicht möglich sind und überschüssiger Sauerstoff entweicht, weil einerseits die Löslichkeit von Sauerstoff in einer $ClO_2^-$-Matrix begrenzt ist, und andererseits sich ein beliebig hoher Sauerstoffpartialdruck nicht aufrechterhalten läßt und auch gar nicht erwünscht ist, da sonst ein erwünschter Peroxidabbau blockiert wird.

Die Anwesenheit von $ClO_2^+$-Ionen, welche sich mit $ClO_3^-$ in einem Gleichgewicht befinden,

$$OClO_2^- + 2\,HOSO_3H \rightleftharpoons ClO_2^+ + 2\,SO_4H^- + H_2O \qquad (3)$$

lösen einen Animpfeffekt aus, der die Reaktion in Richtung von Chlordioxid lenkt :

$$ClO_2^+ + ClO_2^- \longrightarrow 2\,ClO_2 \qquad (4)$$

Der möglichen Bildung von Chlorat wird dadurch entgegengesteuert.

Da das auf diese Weise erhaltene Absorptionsmittel auch Natriumionen enthält, fallen bei der einleitung von $SO_2$ in das Absorptionsmittel nach den vorstehend aufgefühten Reaktionen, d. h. bei der Oxidation zum Sulfat, auf 2 Mol $SO_2$ 2 Mol Natriumsulfat und 1 Mol Natriumchlorid an. Diese beiden Salze lassen sich durch übliche Trennungsverfahren trennen.

Das gewonnene Natriumsulfat kann in Form von Glaubersalz vor Ort, d. h. auf dem Gelände der Großfeuerungsanlage, oder in anderen Bedarfsgegenden als Energiespeicher eingesetzt werden. Bekanntlich kristallisiert Natriumsulfat aus wäßriger Lösung unterhalb 32,38 °C als Dekahydrat (Glaubersalz) in Form farbloser monokliner Prismen aus, während es oberhalb von 32,38 °C als wasserfreies weißes Salz (Thenardit) in Form rhombischer Kristalle ausfällt. Glaubersalz löst sich in Wasser unter Wärmeverbrauch (endotherm), das wasserfreie Salz dagegen unter Wärmeentwicklung (exotherm). Durch die Wahl der Rauchgastemperatur beim Einleiten in die Absorptionsflüssigkeit kann die Temperatur der Absorptionsflüssigkeit und damit die Art der anfallenden Kristallform des Natriumsulfats gesteuert werden.

Die Erfindung wird durch das nachstehende Beispiel näher erläutert ;

Beispiel

A. Herstellung des Absorptionsmittels

In einem geschlossenen Gefäß wurde eine Lösung von 106,4 g (1 Mol) Natriumchlorit in 1 l destilliertem Wasser mit 92,8 ml einer Natriumhypochloritlösung mit einem Gehalt an aktivem Chlor von 13 %, entsprechend 0,21 Mol Natriumhypochlorit versetzt. Diese Lösung, die einen pH-Wert von 10,4 aufwies, wurde unter vorsichtigem Rühren tropfenweise mit Chlorylshwefelsäure entsprechend 6,81 mg ($55{,}6 \times 10^{-6}$ Mol) $KClO_3$ als Katalysator auf einen pH-Wert von 6,4 bis 6,8 titriert. Nach einer Reaktionszeit von 90 Minuten wurde die Lösung vorsichtig mit 6 g (0,039 Mol) Natriumperborat ($NaBO_2 \times H_2O_2 \times 3\ H_3O$) versetzt, und anschließend wurde unter kräftigem Rühren Chlordioxid ausgetrieben. Nach einer kräftigen Reaktion für die Dauer von 15 Minuten wurde das erhaltene Reaktionsgemisch mit 12 g (0,154 Mol) Natriumperoxid ($Na_2O_2$) versetzt. Die dabei auftretende exotherme Reaktion wurde durch stetes Rühren unterbunden. Nach etwa 6 Wochen wurde das Produkt in Form einer wäßrigen Lösung eines Komplexes aus aktiviertem $O_2$ und $ClO_2^-$ von dem ausgefallenen Natriumborat abfiltriert.

Die erhaltene Lösung hatte einen pH-Wert zwischen 13,5 und 13,8, aber keinen Laugencharakter. Sie stellte eine wasserklare, mit Alkoholen mischbare flüssigkeit vom Schmelzpunkt — 3 °c dar. Im Raman-Spektrum wies sie im wesentlichen Banden bei 403, 802 (Chlorit) und 1 562 $cm^{-1}$ (aktivierter Sauerstoff) auf. Zur Bestimmung der zuletzt genannten Bande wurde, da die $O_2$-Bande im Raman-Spektrum teilweise durch die $\delta$ ($OH_2$)-Bande verdeckt wurde, ein Ansatz gemäß vorstehender Arbeitsweise unter Verwendung von $D_2O$ durchgeführt. Im so erhaltenen Konzentrat konnte die $O_2$-Bande eindeutig bei 1 562 $cm^{-1}$ festgelegt werden, da $\delta$ ($OD_2$) wegen des Isotopeneffektes bei niedrigeren Wellenzahlen auftritt. Der O-O-Abstand des sich in der Chlorit-Matrix befindlichen, aktivierten Sauerstoffs wurde zu 123 pm berechnet. Damit war diese Bindung gegenüber dem nichtaktivierten Sauerstoff (120 pm) merklich verlängert.

Mittels Hochdruckflüssigkeitschromatographie ergab sich ein charakteristischer RT-Wert für den Absorptionspeak bei 196 mm von 2,14, der sich deutlich von Peroxid, Chlorit, Hypochlorit und Chlorat unterscheidet.

B. Absorption von $SO_2$

100 mg der in Stufe A erhaltenen Flüssigkeit wurden mit Wasser auf einen pH-Wert von 11,6 verdünnt und als Absorptionsflüssigkeit in einen kleinen Absorberturm gefüllt. Reines Schwefeldioxid wurde aus einer Stahlflasche entnommen und durch eine Heizapparatur geleitet. Dabei wurde das Schwefeldioxid auf 50 °C erwärmt. Anschließend wurden das $SO_2$ mit der Temperatur von 50 °C in den Absorberturm geleitet. Die Reaktion wurde ramanspektroskopisch verfolgt. Dabei wurde festgestellt, daß das $SO_2$ glatt zu Sulfat oxidiert wurde.

Aus der erhaltenen Lösung wurde ein Teil des Natriumsulfats bei Temperaturen oberhalb 32,38 °C als wasserfreies Salz und ein anderer Teil bei Temepraturen unterhalb 32,38 °C als Glaubersalz auskristallisiert.

**Patentansprüche**

1. Verfahren zur Absorption von Schwefeldioxid mittels einer wäßrigen, alkalischen Lösung eines Absorptions-mittels auf Chlorit-Basis, dadurch gekennzeichnet, daß man als Absorptionsmittel eine wäßrige Lösung eines Komplexes aus aktiviertem Sauerstoff und Chlorit mit der allgemeinen Formel $CLO^-_2 \cdot nO_2$, worin n = 0,1 bis 0,25 bedeutet, verwendet.

2. Verfahren nach Anspruch 1, dadurch gekennzeichnet, daß die als Absorptionsmittel verwendete Lösung außerdem Natriumionen enthält und die Absorption unter Temperaturbedingungen durchgeführt wird, bei denen wasserfreies Natriumsulfat oder Glaubersalz entsteht.

3. Verfahren nach einem der Ansprüche 1 oder 2, dadurch gekennzeichnet, daß man als Schwefeldioxidquelle Rauchgas verwendet.

4. Verfahren nach einem der Ansprüche 1 bis 3, dadurch gekennzeichnet, daß die als Absorptionsmittel verwendete Lösung einen pH-Wert von 11,0 bis 14, 5, vorzugsweise von 11,0 bis 13,5 aufweist.

5. Verfahren nach Anspruch 2, dadurch gekennzeichnet, daß man das erhaltene Glaubersalz für Kühlzwecke in Kraftwerken verwendet oder durch Aufheizen mittels Sonnenkollektoren in wasserfreies Natriumsulfat umwandelt.

6. Verfahren nach Anspruch 2 oder 5, dadurch gekennzeichnet, daß man aus dem erhaltenen wasserfreien Natriumsulfat Energie gewinnt.

**Claims**

1. Process for the absorption of sulfur dioxide by an aqueous, alkaline solution of an absorption agent on the basis of chlorite, characterized in that the absorption agent is an aqueous solution of a

complex comprising activated oxygen and chlorite of the general formula $ClO^{-}_2 \cdot nO_2$, wherein n = 0.1 to 0.25.

2. Process according to claim 1, characterized in that the solution used as absorption agent contains also sodium ions and the absorption is carried out at temperature conditions whereby anhydrous sodium sulfate or mirabilite is formed.

3. Process according to one of claims 1 or 2, characterized in that flue gas is used as sulfur dioxide source.

4. Process according to one of claims 1 to 3, characterized in that the solution used as absorption agent has a pH-value of from 11.0 to 14.5, preferably from 11.0 to 13.5.

5. Process according to claim 2, characterized in that the obtained mirabilite is used for cooling purposes in power plants or is converted into anhydrous sodium sulfate by heating with solar collectors.

6. Process according to one of claims 2 or 5, characterized in that energy is produced from the obtained anhydrous sodium sulfate.

**Revendications**

1. Procédé d'absorption d'anhydride sulfureux au moyen d'une solution alcaline aqueuse d'un absorbant à base de chlorite, caractérisé en ce qu'on utilise comme absorbant une solution aqueuse d'un complexe d'oxygène activé et de chlorite de formule générale $ClO^{-}_2 \cdot nO_2$, où n = 0,1 à 0,25.

2. Procédé selon la revendication 1, caractérisé en ce que la solution utilisée comme absorbant contient en outre des ions sodium et en ce que l'absorption est conduite dans des conditions de température où il apparaît du sulfate de sodium anhydre ou du sel de Glauber.

3. Procédé selon l'une des revendications 1 ou 2, caractérisé en ce qu'on utilise comme source d'anhydride sulfureux du gaz de combustion.

4. Procédé selon l'une des revendications 1 à 3, caractérisé en ce que la solution utilisée comme absorbant présente un pH de 11,0 à 14,5 de préférence de 11,0 à 13,5.

5. Procédé selon la revendication 2, caractérisé en ce qu'on utilise le sel de Glauber obtenu dans des buts de refroidissement dans les centrales électriques ou en ce qu'on le transforme en sulfate de sodium anhydre par chauffage au moyen de capteurs solaires.

6. Procédé selon l'une des revendications 2 ou 5, caractérisé en ce qu'on obtient de l'énergie à partir du sulfate de sodium anhydre obtenu.